# EUROPÄISCHE PATENTSCHRIFT

(11) **EP 1 037 586 B1**
(45) Veröffentlichungstag und Bekanntmachung des Hinweises auf die Patenterteilung: **22.12.2004**
(21) Anmeldenummer: 98950106.9
(22) Anmeldetag: 05.10.1998
(51) Int. Cl.: A61K 7/00

(54) **VERWENDUNG VON ONIUMALDEHYDEN UND -KETONEN ZUM FÄRBEN VON KERATINHALTIGEN FASERN**
UTILIZATION OF ONIUM ALDEHYDES AND ONIUM KETONES FOR DYING FIBERS CONTAINING KERATIN
UTILISATION D'ALDEHYDES ET DE CETONES D'ONIUM POUR COLORER DES FIBRES A BASE DE KERATINE

(30) Priorität: 14.10.1997 DE 19745356
(43) Veröffentlichungstag der Anmeldung: 27.09.2000
(73) Patentinhaber: Henkel Kommanditgesellschaft auf Aktien, 40589 Düsseldorf (DE)
(72) Erfinder: MÖLLER, Hinrich, D-40789 Monheim (DE); HÖFFKES, Horst, D-40595 Düsseldorf (DE)
(74) Vertreter: Strohe-Kamp, Geertje
(86) Internationale Anmeldenummer: PCT/EP1998/006308
(87) Internationale Veröffentlichungsnummer: WO 1999/018916

(56) Entgegenhaltungen:
- WO-A-95/01772
- WO-A-97/20545
- DE-A- 19 527 121
- FR-A- 2 757 384
- FR-A- 2 757 388

## Beschreibung

Die Erfindung betrifft die Verwendung von Oniumaldehyden und -ketonen und deren Derivate zum Färben von keratinhaltigen Fasern, insbesondere menschlichen Haaren, ein Mittel zum Färben von keratinhaltigen Fasern, das Oniumaldehyde und -ketone enthält, sowie ein Verfahren zum Färben von keratinhaltigen Fasern.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2'-hydroxyethyl)-N,N'-bis(4'-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ im Vergleich mit üblichen

Oxidationshaarfärbemitteln mindestens gleichwertig sind, ohne jedoch unbedingt auf Oxidationsmittel wie z. B. H₂O₂ angewiesen zu sein. Eine weitere Aufgabe der vorliegenden Erfindung besteht darin, Färbemittel bereitzustellen, mit denen eine große Vielfalt in den Farbnuancen erhalten werden kann. Eine Anfärbung der-Hautpartien sollte möglichst vermieden werden. Darüber hinaus dürfen die Färbemittel kein oder lediglich ein sehr geringes Sensibilisierungspotential aufweisen.

Überraschenderweise wurde gefunden, daß sich eine Kombination aus Oniumaidehyden und -ketonen, wie sie nachstehend definiert werden, und Aminen, Hydroxyverbindungen bzw. CH-aktiven Verbindungen hervorragend zum Färben von keratinhaltigen Fasern eignet. Es werden Ausfärbungen mit hervorragender Brillanz und Farbtiefe in vielfältigen Farbnuancen erzielt. Der Einsatz von oxidierenden Agentien ist nicht erforderlich, er soll jedoch nicht prinzipiell ausgeschlossen werden.

Gegenstand der vorliegenden Erfindung ist demgemäß die Verwendung von Oniumaldehyden und -ketonen der folgenden Formel I oder deren Derivaten 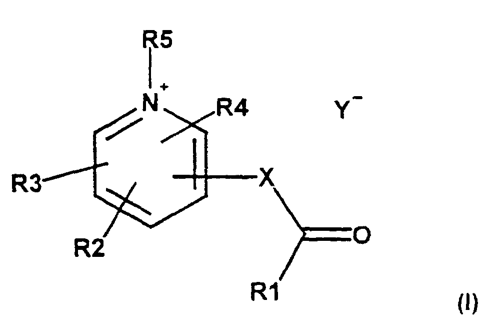 worin R¹ für ein Wasserstoffatom, eine (C₁-C₄)-Alkylgruppe, eine Arylgruppe oder eine Heteroarylgruppe steht,
R², R³ und R⁴ jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Arylgruppe, Trifluormethylgruppe,
Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
R⁵ für eine (C₁-C₄)-Alkylgruppe, Arylgruppe, Aralkylgruppe oder Heteroarylgruppe steht,
X für eine direkte Bindung oder eine Vinylen- oder Phenylengruppe, die substituiert sein kann, steht, und
Y⁻ Halogenid, Benzolsulfonat, p-Toluolsulfonat, Methansulfonat, Trifluormethansulfonat, Perchlorat, Sulfat, Hydrogensulfat oder Tetrachlorzinkat oder das N-Oxid des Heterocyclus bedeutet,
in Kombination mit mindestens einer Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, α- bis ω-Aminosäuren, aus 2 bis 9 Aminosäuren aufgebauten Oligopeptiden und aromatischen Hydroxyverbindungen, und/oder mindestens einer CH-aktiven Verbindung zum Färben von keratinhaltigen Fasern.

Ein weiterer Gegenstand der vorliegenden Erfindung ist ein Mittel zum Färben von keratinhaltigen Fasern, insbesondere menschlichen Haaren, das enthält
(A) ein oder mehrere Oniumaldehyde oder -ketone mit der voranstehend dargestellten Formel I oder deren Derivate und
(B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, α- bis ω-Aminosäuren, aus 2 bis 9 Aminosäuren aufgebauten Oligopeptiden und aromatischen Hydroxyverbindungen, und/oder mindestens eine CH-aktive Verbindung,
wobei auch das Reaktionsprodukt aus den Komponenten A und B enthalten sein kann.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl-
oder Acetylcellulose und synthetischer Fasern, wie z.B. Polyamid-, Polyacrylnitril-, Polyurethan- und Polyesterfasern verwendet werden.

Unter die vorliegende Erfindung fällt auch die Verwendung solcher Substanzen, die Reaktionsprodukte der einzelnen Komponenten untereinander darstellen.

Beispiele für Derivate der Verbindungen mit der Formel I sind Oxime, Acetale, Ketale oder Hydryzone.

Geeignete Verbindungen mit der Formel I, die als Komponente A eingesetzt werden können, sind die Benzolsulfonate, p-Toluolsulfonate, Methansulfonate, Trifluormethansulfonate, Perchlorate, Sulfate, Chloride, Bromide, Jodide und/oder Tetrachlorzinkate von 4-Formyl-1-methylpyridinium, 3-Formyl-1-methylpyridinium, 2-Formyl-1-methylpyridinium, 4-Formyl-1-ethylpyridinium, 2-Formyl-1-ethylpyridinium, 4-Formyl-1-benzylpyridinium, 2-Formyl-1-benzylpyridinium, 4-Formyl-1,2-dimethylpyridinium, 4-Formyl-1,3-dimethylpyridinium, 4-Formyl-1-methylchinolinium, 2-Formyl-1-methylchinolinium, 4-(2-Formylvinyl)-1-methylchinolinium, 4-Acetyl-1-methylpyridinium, 2-Acetyl-1-methylpyridinium, 4-Acetyl-1-chinolinium, 4-Acetyl-1-methylchinolinium und 4-(2-Formylvinyl)-1-methylpyridinium, 2,6-Dichlor-4-formyl-1-methylpryridinium, 2,6-Diphenyl-4-formyl-1-methylpyridinium, 4-Benzoyl-1-methylpyridinium, 4-Propionyl-1-methylpyridinium, 2-Oximomethyl-1-methylpyridinium, 4-Pyridincarboxaldehyd-N-oxid und N-Methylpyridoxal.

Die erfindungsgemäß eingesetzten Verbindungen mit der Formel I sind literaturbekannt und im Handel erhältlich oder lassen sich aus der N-heterocyclischen Carbonylverbindung und einem Alkylierungsmittel in an sich bekannter Weise herstellen. Zur Herstellung der Verbindungen werden die N-heterocyclische Carbonylverbindung und ein Überschuß an Alkylierungsmittel in Toluol gelöst und unter Rühren mehrere Stunden auf 90 bis 100 °C erwärmt, bis die Ausgangsverbindung verschwunden ist. Das Alkylierungsmittel kann in einer Menge bis zum 10- bis 20-fachen Überschuß bezogen auf die N-heterocyclische Carbonylverbindung eingesetzt werden. Während der Reaktion scheidet sich die quartäre Ammoniumverbindung meist harzartig ab. Dieses Harz wird nach Abschluß der Reaktion mehrmals mit heißem Toluol zum vollstänigen Entfernen des Alkylierungsmittels extrahiert und getrocknet. Das Endprodukt ist meist harzartig oder gelegentlich kristallin.

Geeignete Verbindungen mit primärer oder sekundärer Aminogruppe als Komponente B sind -z.B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilindihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, -phenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylamino-, 3-Amino-4-(2'hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor-, 2-Methyl-5-amino-4-chlor-, 3,4-Methylendioxy-, 5-(2-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest, wie sie in der Formel II dargestellt sind 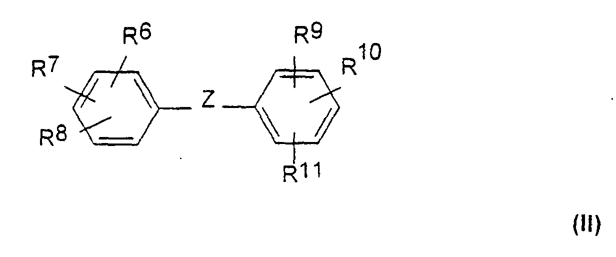 in der R⁶ für eine Hydroxy- oder eine Aminogruppe, die durch C₁₋₄-Alkyl, C₁₋₄-Hydroxyalkyl- oder C₁₋₄-Alkoxy-C₁₋₄-alkyl substituiert sein kann, steht, R⁷, R⁸, R⁹, R¹⁰ und R¹¹ für Wasserstoff, eine Hydroxy- oder eine Aminogruppe, die durch eine C₁₋₄-Alkyl-, C₁₋₄-Hydroxyalkyl, C₁₋₄-Aminoalkyl- oder C₁₋₄-Alkoxy-C₁₋₄-alkylgruppe substituiert sein kann, für eine Carbon- doer Sulfonsäuregruppe stehen, und
Z für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

Q-(CH₂-P-CH₂-Q')ₒ (III)

in der P eine direkte Bindung, eine CH₂- oder CHOH-Gruppe bedeutet,
Q und Q' unabhängig voneinander für ein Sauerstoffatom, eine NR¹²-Gruppe, worin R¹² Wasserstoff, eine C₁₋₄-Alkyl-, oder Hydroxy-C₁₋₄-alkylgruppe bedeutet, die Gruppe
O-(CH₂)ₚ-NH oder NH-(CH₂)ₚ-O, worin p und p' 2 oder 3 sind, stehen und
o eine Zahl von 1 bis 4 bedeutet,
wie beispielsweise 4,4'-Diaminostilben, 4,4'-Diaminostilben-2,2'-disulfonsäure-mono- oder -di-Na-Salz, 4-Amino-4'-dimethylaminostilben, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch. verträglichen Salze, insbesondere als Salze anorganischer Säuren, wie Salz- oder Schwefelsäure, eingesetzt werden.

Geeignete stickstoffhaltige heterocyclische Verbindungen sind z.B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methylpyrimidin, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, -benzothiazol, 2,5-Dihydroxy-4-morpholinoanilin sowie Indole und Indoline, sowie deren physiologisch verträgliche Salze, sein. Bevorzugte Beispiele für Indol- bzw. Indolinderivate 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure. 6-Hydroxyindolin, 6-Aminoindolin und 4-Aminoindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Als Aminosäuren kommen bevorzugt alle natürlich vorkommenden und synthetischen α-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein, zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Lysin und Tryptophan. Es können aber auch andere Aminosäuren verwendet werden, wie z. B. 6- Aminocapronsäure.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Geeignete aromatische Hydroxyverbindungen sind z.B. 2-, 4-, 5-Methylresorcin, 2,5-Dimethylresorcin, Resorcin, 3-Methoxyphenol, Brenzkatechin, Hydrochinon, Pyrogallol; Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als CH-aktive Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat. Rhodanin, Rhodanin-3-essigsäure, 1-Ethyl-2-chinaldiniumiodid, 1-Methyl-2-chinaldiniumiodid Barbitursäure, Thiobarbitursäure, 1,3-Dimethylthiobarbitursäure, Diethylthiobarbitursäure, Oxindol, 3-Indoxylacetat, Cumaranon und 1-Methyl-3-phenyl-2-pyrazolinon.

In allen Färbemitteln können auch mehrere verschiedene färbende Substanzen gemeinsam zum Einsatz komen; ebenso können auch mehrere verschiedene Komponenten aus den Gruppen von Verbindungen mit primärer oder sekundärer Aminogruppe, von stickstoffhaltigen Heterocyclen, aromatischen Hydroxyverbindungen oder Aminosäuren gemeinsam verwendet werden.

Auf die Anwesenheit von Oxidationsmitteln, z.B. H₂O₂, kann bei dererfindungsgemäßen Verwendung der Oniumaldehyde und- ketone der Formel I verzichtet werden. Es kann jedoch u.U. wünschenswert sein, den erfindungsgemäßen Mitteln zur Erzielung von Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

Die erfindungsgemäßen Färbemittel ergeben eine breite Palette von Farbnuancen im Bereich von gelb über gelbbraun, orange, braunorange, mittelbraun, dunkelbraun, violett,
dunkelviolett bis hinzu blauschwarz und schwarz; die Echtheitseigenschaften sind hervorragend, die Sensibilisierungspotentiale sehr gering.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z.B. aus der Gruppe der Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten diedirektziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar
können die Färbemittel üblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind. Falls erforderlich ist es auch möglich, die Färbemittel inwasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Na trium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6-Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO^{(- )}- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie-Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von -Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, VinylpyrrolidonNinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditioniereride Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z.B. -Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Zum Färben der keratinhaltigen Fasern, insbesondere zum Färben von menschlichen Haaren, werden die Färbemittel in der Regel gemeinsam mit einem wasserhaltigen, kosmetischen Trägers in einer Menge von 100 g auf das Haar aufgebracht, ca. 30 Minuten dort belassen und anschließend ausgespült oder mit einem handelsüblichen Haarshampoo ausgewaschen.

Die Verbindungen der Komponenten A und B können entweder gleichzeitig auf das Haar aufgebracht werden oder aber auch nacheinander, wobei es unerheblich ist, welche der Komponenten zuerst aufgetragen wird. Auch der Einsatz der Reaktionsprodukte aus den Komponenten A und B ist möglich. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Komponenten A und B der erfindungsgemäßen Mittel können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (50 bis 80°C) Wassers hinzugefügt und eine homogene Mischung hergestellt.

### Beispiele

### Herstellung der Verbindungen mit der Formel I

0,5 Mol der N-heterocyclischen Carbonylverbindung und 1 Mol Methylsulfat als Alkylierungsmittel wurden in 500 ml Toluol gelöst und unter Rühren 5 Stunden auf 100 °C erwärmt. Während der Reaktion schied sich die quartäre Ammoniumverbindung ab. Das Reaktionsprodukt wurde wird 2 mal mit heißem Toluol extrahiert, um. das Alkylierungsmittel vollständig zu entfernen, und anschließend getrocknet. Das Endprodukt war meist harzartig oder kristallin.

Es wurden erhalten:
4-Acetyl-1-methylpyridiniummethansulfonat: gelblich, harzartig
4-Benzoyl-1-methylpyridiniummethansulfonat: farblos, Schmp. 178 °C
2- und 4-Formyl-1-methylchinoliniummethansulfonat:gelblich, harzarig
4-(2-Formylvinyl)-1-methylpyridinium-trifluormethansulfonat:gelblich, harzartig
4-Acetyl-1-methylchinolinium-methansulfonat: rötlich, harzartig

### Herstellung einer Färbelösung

Es wurde eine Aufschlämmung von 10 mMol einer Färbekomponente, 10 mMol eines Amins, 10 mMol Natriumacetat und einem Tropfen einer 20 %igen Fettalkylethersulfat-Lösung in 100 ml Wasser bereitet. Die Aufschlämmung wurde kurz auf ca. 80°C erhitzt und nach dem Abkühlen filtriert, der pH-Wert wurde anschließend auf 6 eingestellt.

In diese Färbelösung wurde bei 30°C 30 Minuten lang eine Strähne zu 90% ergrauten, nicht vorbehandelten Menschenhaares eingebracht. Die gefärbte Strähne wurde anschließend 30 Sek. mit lauwarmem Wasser gespült, im warmen (30-40°C) Luftstrom getrocknet und anschließend ausgekämmt. Danach wurden die Ausfärbungen visuell bei Tageslicht beurteilt.

Die jeweiligen Farbnuancen und Farbtiefen sind in der nachfolgenden Tabellen wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+)- :: starke bis sehr starke Intensität
- +++ :: sehr starke Intensität

**Tabelle 1**

| **Ausfärbungen mit 4-Formyl-1-methyl-pyridinium-benzolsulfonat** | | |
|---|---|---|
| **Komponente B** | **Färbenuance** | |
| | **Farbtiefe** | |
| - | - | - |
| 2,5-Diaminotoluol x H₂SO₄ | orangerot | |
| ++(+) | | |
| 2,4,5,6-Tetraaminopyrimidin x H₂SO₄ | braunorange | ++ |
| 1,8-Bis-(2,5-diaminophenoxy)-3,6-dioxaoctan x 4 HCl | orangebraun | ++ |
| 2-Methylamino-3-amino-6-methoxypyridin x 2HCl | dunkelviolett | +++ |
| 2-(2,5-Diaminophenyl)-ethanol x H₂SO₄ | orangerot | |
| ++(+) | | |
| 2-Aminomethyl-4-aminophenol x 2 HCl | gelbolivbraun | ++ |
| N,N-Bis-(2-hydroxyethyl)-p-phenylendiamin x HCl | violettrot | |
| | ++(+) | |
| 4,4'-Diaminodiphenylamin x H₂SO₄ | schwarzviolett | +++ |
| 2,6-Dimethoxy-3,5-diaminopyridin x 2HCl | dunkelbraun | +++ |
| Fischersche Base | violettrot | ++ |

**Tabelle 2**

| **Ausfärbungen mit 4-Acetyl-1-methylpyridinium-methansulfonat** | | |
|---|---|---|
| **Komponente B** | **Färbenuance** | |
| | **Farbtiefe** | |
| - | - | - |
| 2,5-Diaminotoluol x H₂SO₄ | rotbraun | ++ |
| 2,4,5,6-Tetraaminopyrimidin x H₂SO₄ | braunrot | ++ |
| 1,8-Bis-(2,5-diaminophenoxy)-3,6-dioxaoctan x 4 HCl | dunkelblaugrau | |
| ++(+) | | |
| 2-Methylamino-3-amino-6-methoxypyridin x 2HCl | violettbraun | +++ |
| 2-(2,5-Diaminophenyl)-ethanol x H₂SO₄ | rotbraun | +(+) |
| 2-Aminomethyl-4-aminophenol x 2 HCl | gelbbraun | +(+) |
| N,N-Bis-(2-hydroxyethyl)-p-phenylendiamin x HCl | violettbraun | ++ |
| 4,4'-Diaminodiphenylamin x H₂SO₄ | schwarz | +++ |
| 2,6-Dimethoxy-3,5-diaminopyridin x 2HCl | schwarz | +++ |

**Tabelle 3**

| **Ausfärbungen mit 4-Benzoyl-1-methylpyridinium-methansulfonat** | | |
|---|---|---|
| **Komponente B** | **Färbenuance** | |
| | **Farbtiefe** | |
| - | - | - |
| 2,5-Diaminotoluol x H₂SO₄ | dunkelviolett | +++ |
| 2,4,5,6-Tetraaminopyrimidin x H₂SO₄ | orange | ++ |
| 1,8-Bis-(2,5-diaminophenoxy)-3,6-dioxaoctan x 4 HCl | blauschwarz | +++ |
| 2-Methylamino-3-amino-6-methoxypyridin x 2HCl | schwarzbraun | +++ |
| 2-(2,5-Diaminophenyl)-ethanol x H₂SO₄ | rotbraun | ++ |
| 2-Aminomethyl-4-aminophenol x 2 HCl | mittelbraun | +(+) |
| N,N-Bis-(2-hydroxyethyl)-p-phenylendiamin x HCl | violettbraun | ++ |
| 4,4'-Diaminodiphenylamin x H₂SO₄ | schwarz | +++ |
| 2,6-Dimethoxy-3,5-diaminopyridin x 2HCl | schwarz | +++ |

**Tabelle 4**

| **Ausfärbungen mit 2-Oximomethyl-1-methylpyridinium-methansulfonat** | | |
|---|---|---|
| **Komponente B** | **Färbenuance** | |
| | **Farbtiefe** | |
| - | - | - |
| 2,5-Diaminotoluol x H₂SO₄ | violettbraun | |
| ++(+) | | |
| 2,4,5,6-Tetraaminopyrimidin x H₂SO₄ | orange | |
| ++(+) | | |
| 1,8-Bis-(2,5-diaminophenoxy)-3,6-dioxaoctan x 4 HCl | blauschwarz | +++ |
| 2-Methylamino-3-amino-6-methoxypyridin x 2HCl | dunkelbraun | +++ |
| 2-(2,5-Diaminophenyl)-ethanol x H₂SO₄ | violettbraun | ++ |
| 2-Aminomethyl-4-aminophenol x 2 HCl | hellbraun | + |
| N,N-Bis-(2-hydroxyethyl)-p-phenylendiamin x HCl | mittelbraun | ++ |
| 4,4'-Diaminodiphenylamin x H₂SO₄ | blauschwarz | +++ |
| 2,6-Dimethoxy-3,5-diaminopyridin x 2HCl | schwarz | +++ |

**Tabelle 5**

| **Ausfärbungen mit 2-Formyl-1-methylchinolinium-triflourmethansulfonat** | | |
|---|---|---|
| **Komponente B** | **Färbenuance** | |
| | **Farbtiefe** | |
| - | hellbraun | + |
| 2,5-Diaminotoluol x H₂SO₄ | blauschwarz | +++ |
| 2,4,5,6-Tetraaminopyrimidin x H₂SO₄ | violettrot | |
| ++(+) | | |
| 1,8-Bis-(2,5-diaminophenoxy)-3,6-dioxaoctan x 4 HCl | blauschwarz | +++ |
| 2-Methylamino-3-amino-6-methoxypyridin x 2HCl | schwarz | +++ |
| 2-(2,5-Diaminophenyl)-ethanol x H₂SO₄ | violettschwarz | +++ |
| 2-Aminomethyl-4-aminophenol x 2 HCl | schwarz | +++ |
| N,N-Bis-(2-hydroxyethyl)-p-phenylendiamin x HCl | blauschwarz | +++ |
| 4,4'-Diaminodiphenylamin x H₂SO₄ | blauschwarz | +++ |
| 2,6-Dimethoxy-3,5-diaminopyridin x 2HCl | blauschwarz | +++ |
| 3-Methyl-4-aminophenol (Oxyrot) | rot | ++ |

**Tabelle 6**

| **Ausfärbungen mit 4-Formyl-1-methylchinolinium-methylsulfat** | | |
|---|---|---|
| **Komponente B** | **Färbenuance** | |
| | **Farbtiefe** | |
| 2,5-Diaminotoluol x H₂SO₄ | dunkelviolett | +++ |
| 2-Methylamino-3-amino-6-methoxypyridin x 2HCl | dunkelblau | +++ |
| 2-(2,5-Diaminophenyl)-ethanol x H₂SO₄ | dunkelviolett | +++ |
| 2-Aminomethyl-4-aminophenol x 2 HCl | dunkelbraun | ++ |
| N,N-Bis-(2-hydroxyethyl)-p-phenylendiamin x HCl | dunkelblau | +++ |
| 2,6-Dimethoxy-3,5-diaminopyridin x 2HCl | dunkelbraun | +++ |

**Tabelle 7**

| **Ausfärbungen mit 4-Formyl-1-methylchinolinium-methansulfonat** | | |
|---|---|---|
| **Komponente B** | **Färbenuance** | |
| | **Farbtiefe** | |
| | rosa | + |
| 2,5-Diaminotoluol x H₂SO₄ | braunviolett | +++ |
| 2,4,5,6-Tetraaminopyrimidin x H₂SO₄ | dunkel-violettrot | +++ |
| 1,8-Bis-(2,5-diaminophenoxy)-3,6-dioxaoctan x 4 HCl | rotviolett | +++ |
| 2-Methylamino-3-amino-6-methoxypyridin x 2HCl | blauschwarz | +++ |
| 2-(2,5-Diaminophenyl)-ethanol x H₂SO₄ | dunkelviolett | +++ |
| 2-Aminomethyl-4-aminophenol x 2 HCl | orangebraun | ++ |
| N,N-Bis-(2-hydroxyethyl)-p-phenylendiamin x HCl | schwarzblau | +++ |
| 4,4'-Diaminodiphenylamin x H₂SO₄ | schwarzblau | +++ |
| 2,6-Dimethoxy-3,5-diaminopyridin x 2HCl | dunkelbraun | +++ |
| 3,4-Diaminobenzoesäure | dunkelviolett | |
| ++(+) | | |
| 4-Hydroxy-2,5,6-triamino-pyrimidin-sulfat | braunrosa | +(+) |
| Ausfärbungen mit dem Reaktionsprodukt aus den Komponenten A und B | | |
| Komponente A: 4-Formyi-1-methylchinolinium-methansulfonat | | |
| Komponente B: N,N-Bis-(2-hydroxyethyl)-p-phenylendiamin x HCl | | |
| Farbnuance: schwarzblau, Farbtiefe +++ | | |

**Tabelle 8**

| **Ausfärbungen mit 4-(2-Formylvinyl)-1-methylpyridinium-trifluormethansulfonat** | | |
|---|---|---|
| **Komponente B** | **Färbenuance** | |
| | **Farbtiefe** | |
| - | braungelb | + |
| 2,5-Diaminotoluol x H₂SO₄ | violettrot | |
| ++(+) | | |
| 2-Methylamino-3-amino-6-methoxypyridin x 2HCl | rotschwarz | +++ |
| 2-(2,5-Diaminophenyl)-ethanol x H₂SO₄ | rot | |
| ++(+) | | |
| 2-Aminomethyl-4-aminophenol x 2 HCl | braungelb/messing | ++ |
| 4,4'-Diaminodiphenylamin x H₂SO₄ | schwarzviolett | +++ |
| 2,6-Dimethoxy-3,5-diaminopyridin x 2HCl | blauschwarz | +++ |

**Tabelle 9**

| **Ausfärbungen mit 4-Acetylchinolinium-methansulfonat** | | |
|---|---|---|
| **Komponente B** | **Färbenuance** | |
| | **Farbtiefe** | |
| 2,5-Diaminotoluol x H₂SO₄ | rotbraun | ++ |
| 2-(2,5-Diaminophenyl)-ethanol x H₂SO₄ | rotbraun | ++ |
| 2-Aminomethyl-4-aminophenol x 2 HCl | orangebraun | +(+) |
| N,N-Bis-(2-hydroxyethyl)-p-phenylendiamin x HCl | dunkelbraun | |
| | ++(+) | |
| 2-Amino-4-(2-hydroyethylamino)-anisol x H₂SO₄ | | |
| (Lehmann's blau) | grauschwarz | |
| ++(+) | | |
| 3-Methyl-p-amino-phenol | orangebraun | +(+) |
| 5-Amino-o-kresol | rotbraun | ++ |

**Tabelle 10**

| **Ausfärbungen mit 4-Pyridincarboxaldehyd-N-oxid** | | |
|---|---|---|
| **Komponente B** | **Färbenuance** | |
| | **Farbtiefe** | |
| 2,4,5,6-Tetraaminopyrimidin x H₂SO₄ | gelb | ++ |
| 2-Methylamino-3-amino-6-methoxypyridin x 2HCl | orangebraun | ++ |
| N,N-Bis-(2-hydroxyethyl)-p-phenylendiamin x HCl | gelbbraun | ++ |

## Patentansprüche

1. Verwendung von Oniumaldehyden und -ketonen der folgenden Formel I oder deren Derivaten 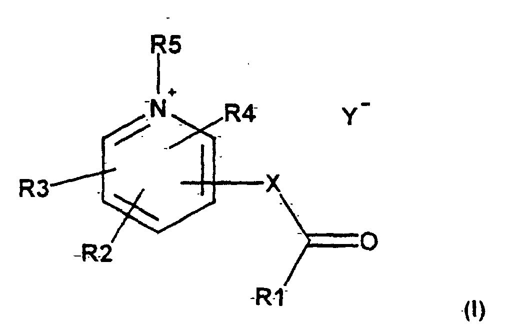 worin
R¹ für ein Wasserstoffatom, eine (C₁-C₄)-Alkylgruppe, eine Arylgruppe oder eine Heteroarylgruppe steht,
R², R³ und R⁴ jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Arylgruppe, Trifluormethylgruppe, Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
R⁵ für eine (C₁-C₄)-Alkylgruppe, Arylgruppe, Aralkylgruppe oder Heteroarylgruppe steht,
X für eine direkte Bindung oder eine ggf. substituierte Vinylen- oder Phenylengruppe steht, und
Y⁻ Halogenid, Benzolsulfonat, p-Toluolsulfonat, Methansulfonat, Trifluormethansulfonat, Perchlorat, Sulfat, Hydrogensulfat, Tetrachlorzinkat oder N-Oxid des Heterocyclus bedeutet,
in Kombination mit mindestens einer Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, α- bis ω-Aminosäuren, aus 2 bis 9 Aminosäuren aufgebauten Oligopeptiden und aromatischen Hydroxyverbindungen, und/oder mindestens einer CH-aktiven Verbindung zum Färben von keratinhaltigen Fasern.

2. Mittel zum Färben von keratinhaltigen Fasern, insbesondere menschlichen Haaren, das enthält
(A) ein oder mehrere Oniumaldehyde und -ketone mit der folgenden Formel I oder deren Derivate 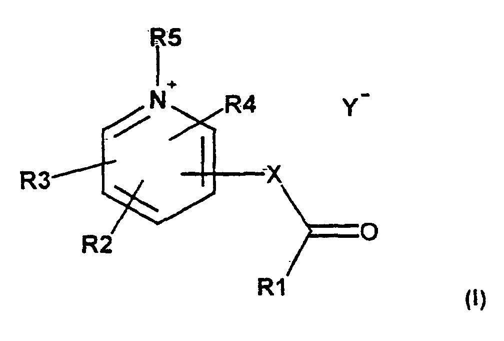 worin
R¹ für ein Wasserstoffatom, eine (C₁-C₄)-Alkylgruppe, eine Arylgruppe oder eine Heteroarylgruppe steht,
R², R ³ und R⁴ jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Arylgruppe, Trifluormethylgruppe, Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
R⁵ für eine (C₁-C₄)-Alkylgruppe, Arylgruppe, Aralkylgruppe oder Heteroarylgruppe steht,
X für eine direkte Bindung oder eine ggf. substituierte Vinylen- oder Phenylengruppe steht, und
Y⁻ Halogenid, Benzolsulfonat, p-Toluolsulfonat, Methansulfonat, Trifluormethansulfonat, Perchlorat, Sulfat, Hydrogensulfat, Tetrachlorzinkat oder N-Oxid des Heterocyclus bedeutet, und
(B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, α- bis ω-Aminosäuren, aus 2 bis 9 Aminosäuren aufgebauten Oligopeptiden und aromatischen Hydroxyverbindungen, und/oder mindestens eine CH-aktive Verbindung,
wobei auch das Reaktionsprodukt aus den Komponenten A und B enthalten sein kann.

3. Mittel nach Anspruch 2, **dadurch gekennzeichnet, daß** die Komponente A ausgewählt ist der Gruppe bestehend aus den Benzolsulfonaten, p-Toluolsulfonaten, Methansulfonaten, Trifluormethansulfonaten, Perchloraten, Sulfaten, Chloriden, Bromiden, Jodiden und/oder Tetrachlorzinkaten von 4-Formyl-1-methylpyridinium, 3-Formyl-1-methylpyridinium, 2-Formyl-1-methylpyridinium, 4-Formyl-1-ethylpyridinium, 2-Formyl-1-ethylpyridinium, 4-Formyl-1-benzylpyridinium, 2-Formyl-1-benzylpyridinium, 4-Formyl-1,2-dimethylpyridinium, 4-Formyl-1,3-dimethylpyridinium, 4-Formyl-1-methylchinolinium, 2-Formyl-1-methylchinolinium, 4-(2-Formylvinyl)-1-methylchinolinium, 4-Acetyl-1-methylpyridinium, 2-Acetyl-1-methylpyridinium, 4-Acetyl-1-chinolinium, 4-Acetyl-1-methylchinolinium und 4-(2-Formylvinyl)-1-methylpyridinium, 2,6-Dichlor-4-formyl-1-methylpryridinium, 2,6-Diphenyl-4-formyl-1-methylpyridinium, 4-Benzoyl-1-methylpyridinium, 4-Propionyl-1-methylpyridinium, 2-Oximomethyl-1-methylpyridinium, 4-Pyridincarboxaldehyd-N-oxid und N-Methylpyridoxal.

4. Mittel nach Anspruch 2 oder 3, **dadurch gekennzeichnet, daß** die Verbindungen der Komponente B ausgewählt sind aus primären oder sekundären Aminen aus der Gruppe, bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethyl)-pphenylendiamin, 2,5-Dihydroxy-4-morpholinoanilin-dihydrobromid, 2-, 3-, 4-Aminophenol, o-, p-Phenylendiamin, 2,4-Diaminophenoxyethanol, 2-(2,5-Diaminophenyl)-ethanol, 2,5-Diaminotoluol, -phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-,3,4-Methylendioxy-, 3-Methyl-4-amino-, 2-Methyl-5-(2-hydroxyethylamino)-, 6-Methyl-3-amino-2-chlor-, 6-Methyl-3-amino-2-chlor-, 5-(2-Hydroxyethylamino)-4-methoxy-2-methyl-, 4-Amino-2-aminomethylphenol, 4-Amino-2-hydroxymethyl-phenol, 3,4-Methylendioxyphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Ami-nophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy)-propan-tetrahydrochlorid, 1,8-Bis-(2,5-diaminophenoxy)-3,6-dioxaoctan-tetrahydrochlorid, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]-methylamin-trihydrochlorid, stickstoffhaltigen heterocyclischen Verbindungen aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-6-Aminoindazol, 5-, 7-Amino-benzimidazol, -benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin und 4-Hydroxyindolin, sowie jeweils aus den mit vorzugsweise anorganischen Säuren gebildeten physiologisch verträglichen Salzen dieser Verbindungen, aromatischen Hydroxyverbindungen aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, - acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure,3,6-Dihydroxy-2,7-naphthalinsulfonsäure, und CH-aktiven Verbindungen aus der Gruppe, bestehend aus 1,2,3,3-Tetramethyl-3H-indoliumiodid, 1,2,3,3-Tetramethyl-3-indolium-p-toluolsulfonat, 1,2,3,3-Tetramethyl-3-indoliummethansulfonat, 2,3-Dimethyl-benzothiazoliumiodid, 2,3-Dimethyl-benzothiazolium-p-toluolsulfonat, Rhodanin, Rhodanin-3-essigsäure, 1-Ethyl(Methyl)-2-chinaldiniumiodid, Barbitursäure, Thiobarbitursäure, 1,3-Dimethyl(ethyl)thiobarbitursäure, Oxindol, Cumaranon und 1-Methyl-3-phenyl-2-pyrazolinon.

5. Mittel nach einem der Ansprüche 2 bis 4, **dadurch gekennzeichnet, daß** die Verbindungen der Komponenten A und B in einer Menge von jeweils 0,03 bis 65, insbesondere 1 bis 40 mmol, jeweils bezogen auf 100 g des gesamten Färbemittels, enthalten sind.

6. Mittel nach einem der Ansprüche 2 bis 5, **dadurch gekennzeichnet, daß** Oxidationsmittel in einer Menge von 0,01 bis 6 Gew.-%, bezogen auf die Anwendungslösung, eingesetzt werden.

7. Mittel nach einem der Ansprüche 2 bis 6, **dadurch gekennzeichnet, daß** als Oxidationsmittel H₂O₂ eingesetzt wird.

8. Mittel nach einem der Ansprüche 2 bis 7, **dadurch gekennzeichnet, daß** anionische, zwitterionische und/oder nichtionische Tenside eingesetzt werden.

9. Verfahren zum Färben von keratinhaltigen Fasern, worin ein Färbemittel, enthaltend
(A) ein oder mehrere Oniumaldehyde und -ketone mit der folgenden Formel I oder deren Derivate 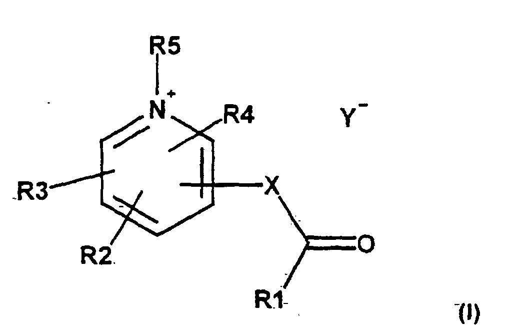 worin
R¹ für ein- Wasserstoffatom, eine (C₁-C₄)-Alkylgruppe, eine Arylgruppe oder eine Heteroarylgruppe steht,
R², R ³ und R⁴ jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Arylgruppe, Trifluormethylgruppe, Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
R⁵ für eine (C₁-C₄)-Alkylgruppe, Arylgruppe, Aralkylgruppe oder Heteroarylgruppe steht,
X für eine direkte Bindung oder eine ggf. substituierte Vinylen- oder Phenylengruppe steht, und
Y⁻ Halogenid, Benzolsulfonat, p-Toluolsulfonat, Methansulfonat, Trifluormethansulfonat, Perchlorat, Sulfat, Hydrogensulfat, Tetrachlorzinkat oder N-Oxid des Heterocyclus bedeutet, und
(B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung,
wobei auch das Reaktionsprodukt aus den Komponenten A und B enthalten sein kann,
sowie übliche kosmetische Inhaltsstoffe auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. The use of onium aldehydes and ketones corresponding to formula I below or derivatives thereof: 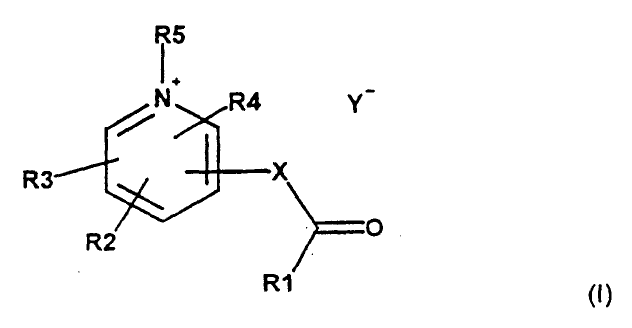 in which
R¹ is a hydrogen atom, a (C₁₋₄) alkyl group, an aryl group or a heteroaryl group,
R², R³ and R⁴ independently of one another represent a hydrogen atom, a halogen atom, a (C₁₋₄) alkyl group, a (C₁₋₄) alkoxy group, hydroxy-(C₁₋₄)-alkoxy group, hydroxy group, nitro group, aryl group, trifluoromethyl group, amino group which may be substituted by (C₁₋₄) alkyl groups or a (C₁₋₄) acyl group; two of the substituents together may also form a fused benzene ring,
R⁵ is a (C₁₋₄) alkyl group, aryl group, aralkyl group or heteroaryl group,
X is a direct bond or a vinylene or phenylene group which may be substituted and
Y⁻ is halide, benzenesulfonate, p-toluene sulfonate, methane sulfonate, trifluoromethane sulfonate, perchlorate, sulfate, hydrogen sulfate or tetrachlorozincate or the N-oxide of the heterocycle,
in combination with at least one compound containing a primary or secondary amino group or hydroxy group selected from primary or secondary aliphatic or aromatic amines, nitrogen-containing heterocyclic compounds, α- to ω-amino acids, oligopeptides made up of 2 to 9 amino acids and aromatic hydroxy compounds and/or at least one CH-active compound,
for coloring keratin-containing fibers.

2. A composition for coloring keratin-containing fibers, more particularly human hair, containing
(A) one or more onium aldehydes and ketones corresponding to formula I or derivatives thereof: 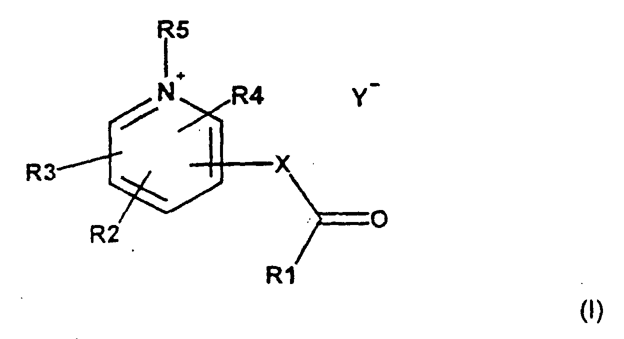 in which
R¹ is a hydrogen atom, a (C₁₋₄) alkyl group, an aryl group or a heteroaryl group,
R², R³ and R⁴ independently of one another represent a hydrogen atom, a halogen atom, a (C₁₋₄) alkyl group, a (C₁₋₄) alkoxy group hydroxy-(C₁₋₄)-alkoxy group, hydroxy group, nitro group, aryl group, trifluoromethyl group, amino group which may be substituted by (C₁₋₄) alkyl groups or a (C₁₋₄) acyl group; two of these substituents together may also form a fused benzene ring,
R⁵ is a (C₁₋₄) alkyl group, aryl group, aralkyl group or heteroaryl group,
X is a direct bond or a vinylene or phenylene group which may be substituted and
Y⁻ is halide, benzenesulfonate, p-toluene sulfonate, methane sulfonate, trifluoromethane sulfonate, perchlorate, sulfate, hydrogen sulfate or tetrachlorozincate or the N-oxide of the heterocycle,
and
(B) at least one compound containing a primary or secondary amino group or hydroxy group selected from primary or secondary aliphatic or aromatic amines, nitrogen-containing heterocyclic compounds, α- to ω-amino acids, oligopeptides made up of 2 to 9 amino acids and aromatic hydroxy compounds and/or at least one CH-active compound; the reaction product of components A and B may also be present.

3. A composition as claimed in claim 2, **characterized in that** component A is selected from the group consisting of benzenesulfonates, p-toluene sulfonates, methane sulfonates, trifluoromethane sulfonates, perchlorates, sulfates, chlorides, bromides, iodides and/or tetrachlorozincates of 4-formyl-1-methyl pyridinium, 3-formyl-1-methyl pyridinium, 2-formyl-1-methyl pyridinium, 4-formyl-1-ethyl pyridinium, 2-formyl-1-ethyl pyridinium, 4-formyl-1-benzyl pyridinium, 2-formyl-1-benzyl pyridinium, 4-formyl-1,2-dimethyl pyridinium, 4-formyl-1,3-dimethyl pyridinium, 4-formyl-1-methyl quinolinium, 2-formyl-1-methyl quinolinium, 4-(2-formylvinyl)-1-methyl quinolinium, 4-acetyl-1-methyl pyridinium, 2-acetyl-1-methyl pyridinium, 4-acetyl-1-quinolinium, 4-acetyl-1-methyl quinolinium and 4-(2-formylvinyl)-1-methyl pyridinium, 2,6-dichloro-4-formyl-1-methyl pyridinium, 2,6-diphenyl-4-formyl-1-methyl pyridinium, 4-benzoyl-1-methyl pyridinium, 4-propionyl-1-methyl pyridinium, 2-oximomethyl-1-methyl pyridinium, 4-pyridine carboxaldehyde-N-oxide and N-methyl pyridoxal.

4. A composition as claimed in claim 2 or 3, **characterized in that** the compounds of component B are selected from primary or secondary amines from the group consisting of N-(2-hydroxyethyl)-N-ethyl-, N-(2-methoxyethyl)-, 2,3-, 2,4-, 2,5-dichloro-p-phenylenediamine, 2-chloro-p-phenylenediamine, N,N-bis-(2-hydroxyethyl)-p-phenylenediamine, 2,5-dihydroxy-4-morpholinoaniline dihydrobromide, 2-, 3-, 4-aminophenol, o-, p-phenylenediamine, 2,4-diaminophenoxyethanol, 2-(2,5-diaminophenyl)-ethanol, 2,5-diaminotoluene, -phenol, -phenethol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 3,4-methylenedioxy-, 3-methyl-4-amino-, 2-methyl-5-(2-hydroxyethylamino)-, 6-methyl-3-amino-2-chloro-, 5-(2-hydroxyethylamino)-4-methoxy-2-methyl-, 4-amino-2-aminomethyl phenol, 4-amino-2-hydroxymethyl phenol, 3,4-methylenedioxyphenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-amino-benzenesulfonic acid, 3-amino-4-hydroxybenzenesulfonic acid, 4-amino-3-hydroxynaphthalene sulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechol sulfate, 4,6-diaminopyrogallol dihydrochloride, 3,5-diamino-4-hydroxypyrocatechol sulfate, aromatic anilines and phenols containing another aromatic radical, such as 4,4'-diaminostilbene dihydrochloride, 4,4'-diaminostilbene-2,2'-disulfonic acid sodium salt, 4,4'-diaminodiphenyl methane, -sulfide, -sulfoxide, -amine, 4,4'-diaminodiphenylamine-2-sulfonic acid, 4,4'-diaminobenzophenone, -diphenyl ether, 3,3',4,4'-tetraaminodiphenyl tetrahydrochloride, 3,3'4,4'-tetraaminobenzophenone, 1,3-bis-(2,4-diaminophenoxy)-propane tetrahydrochloride, 1,8-bis-(2,5-diaminophenoxy)-3,6-dioxaoctane tetrahydrochloride, 1,3-bis-(4-aminophenylamino)-propane, -2-propanol, 1,3-bis-[N-(4-aminophenyl)-2-hydroxyethylamino]-2-propanol, N,N-bis-[2-(4-aminophenoxy)-ethyl]-methylamine trihydrochloride;
nitrogen-containing heterocyclic compounds selected from the group consisting of 2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethyl pyridine, 4,5,6-triamino-, 2-hydroxy-4,5,6-triamino-, 4-hydroxy-2,5,6-triamino-, 2,4,5,6-tetraamino-, 2-methylamino-4,5,6-triamino-, 2,4-, 4,5-diamino-, 2-amino-4-methoxy-6-methyl pyrimidine, 2,3,4-trimethyl pyrrole, 2,4-dimethyl-3-ethyl pyrrole, 3,5-diaminopyrazole, -1,2,4-triazole, 3-amino-, 3-amino-5-hydroxypyrazole, 2-, 3-, 8-aminoquinoline, 4-aminoquinaldine, 2-, 6-aminonicotinic acid, 5-aminoisoquinoline, 5-, 6-aminoindazole, 5-, 7-aminobenzimidazole, - benzothiazole, 2,5-dihydroxy-4-morpholinoaniline and indole and indoline derivatives, such as 4-, 5-, 6-, 7-aminoindole, 4-, 5-, 6-, 7-hydroxyindole, 5,6-dihydroxyindole, 5,6-dihydroxyindoline and 4-hydroxyindoline and physiologically compatible salts of these compounds formed with preferably inorganic acids;
aromatic hydroxy compounds selected from the group consisting of 2-, 4-, 5-methyl resorcinol, 2,5-dimethyl resorcinol, resorcinol, 3-methoxyphenol, pyrocatechol, hydroquinone, pyrogallol, phloroglucinol, hydroxyhydroquinone, 2-, 3-, 4-methoxy-, 3-dimethylamino-, 2-(2-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalene sulfonic acid, 3,6-dihydroxy-2,7-naphthalene sulfonic acid and
CH-active compounds selected from the group consisting of 1,2,3,3-tetramethyl-3H-indolium iodide, 1,2,3,3-tetraamethyl indolium-p-toluene sulfonate, 1,2,3,3-tetramethyl-3-indolium methane sulfonate, 2,3-dimethylbenzothiazolium iodide, 2,3-dimethylbenzothiazolium-p-toluene sulfonate, rhodanine, rhodanine-3-acetic acid, 1-ethyl(methyl)-2-quinaldinium iodide, barbituric acid, thiobarbituric acid, 1,3-dimethyl(ethyl) thiobarbituric acid, oxindole, coumaranone and 1-methyl-3-phenyl-2-pyrazolinone.

5. A composition as claimed in any of claims 2 to 4, **characterized in that** the compounds of components A and B are each present in a quantity of 0.03 to 65 mmol and more particularly 1 to 40 mmol, based on 100 g of the colorant as a whole.

6. A composition as claimed in any of claims 2 to 5, **characterized in that** oxidizing agents are used in a quantity of 0.01 to 6% by weight, based on the solution applied.

7. A composition as claimed in any of claims 2 to 6, **characterized in that** H₂O₂ is used as the oxidizing agent.

8. A composition as claimed in any of claims 2 to 7, **characterized in that** anionic, zwitterionic and/or nonionic surfactants are used.

9. A process for coloring keratin-containing fibers in which a colorant containing
(A) one or more onium aldehydes and ketones corresponding to formula I or derivatives thereof: 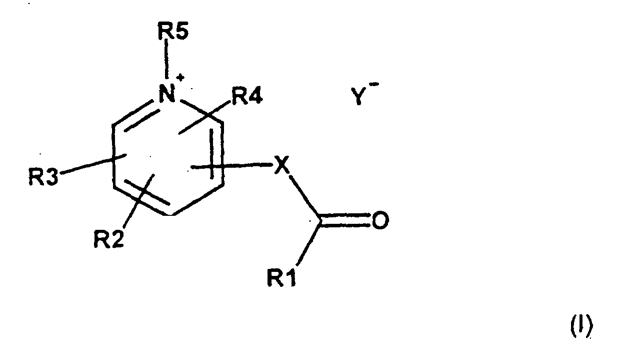 in which
R¹ is a hydrogen atom, a (C₁₋₄) alkyl group, an aryl group or a heteroaryl group,
R², R³ and R⁴ independently of one another represent a hydrogen atom, a halogen atom, a (C₁₋₄) alkyl group, a (C₁₋₄) alkoxy group hydroxy-(C₁₋₄)-alkoxy group, hydroxy group, nitro group, aryl group, trifluoromethyl group, amino group which may be substituted by (C₁₋₄) alkyl groups or a (C₁₋₄) acyl group; two of these substituents together may also form a fused benzene ring,
R⁵ is a (C₁₋₄) alkyl group, aryl group, aralkyl group or heteroaryl group,
X is a direct bond or a vinylene or phenylene group which may be substituted and
Y⁻ is halide, benzenesulfonate, p-toluene sulfonate, methane sulfonate, trifluoromethane sulfonate, perchlorate, sulfate, hydrogen sulfate or tetrachlorozincate or the N-oxide of the heterocycle,
and
(B) at least one compound containing a primary or secondary amino group or hydroxy group selected from primary or secondary aliphatic or aromatic amines, nitrogen-containing heterocyclic compounds, α-to ω-amino acids, oligopeptides made up of 2 to 9 amino acids and aromatic hydroxy compounds and/or at least one CH-active compound; the reaction product of components A and B may also be present,
and typical cosmetic ingredients is applied to the keratin-containing fibers, left thereon for a time, usually about 30 minutes, and then rinsed out again or washed out with a shampoo.

## Revendications

1. Utilisation d'aldéhydes et de cétones à radicaux onium répondant à la formule I ci-après ou de leurs dérivés 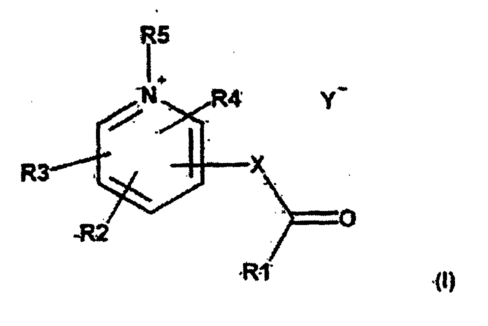 dans laquelle
R¹ représente un atome d'hydrogène, un groupe alkyle en C₁-C₄, un groupe aryle ou un groupe hétéroaryle,
R², R³ et R⁴ représentent, chaque fois indépendamment l'un de l'autre, un atome d'hydrogène, un atome d'halogène, un groupe alkyle en C₁-C₄, un groupe alcoxy en C₁-C₄, un groupe hydroxyalcoxy en C₁-C₄, un groupe hydroxyle, un groupe nitro, un groupe aryle, un groupe trifluorométhyle, un groupe amino qui peut être substitué par des groupes alkyle en C₁-C₄, ou un groupe acyle en C₁-C₄, deux des radicaux pouvant également former ensemble un noyau benzénique condensé,
R⁵ représente un groupe alkyle en C₁-C₄, un groupe aryle, un groupe aralkyle ou un groupe hétéroaryle,
X représente une liaison directe ou un groupe vinylène ou phénylène éventuellement substitué, et
Y⁻ représente un halogénure, un benzènesulfonate, un p-toluènesulfonate, un méthane-sulfonate, un trifluorométhane-sulfonate, un perchlorate, un sulfate, un hydrogénosulfate, un tétrachlorozincate ou un N-oxyde du groupe hétérocyclique,
en combinaison avec au moins un composé comprenant un groupe amino primaire ou secondaire ou un groupe hydroxyle, choisi parmi le groupe comprenant des amines primaires ou secondaires aliphatiques ou aromatiques, des composés hétérocycliques azotés, des acides α- à ω-aminés, des oligopeptides constitués par un nombre de 2 à 9 acides aminés, et des composés hydroxylés aromatiques, et/ou au moins un composé contenant un groupe CH actif pour la teinture de fibres kératiniques.

2. Agent pour la teinture de fibres kératiniques, en particulier de cheveux humains, qui contient
(A) un ou plusieurs aldéhydes et une ou plusieurs cétones à radicaux onium répondant à la formule I ci-après ou leurs dérivés 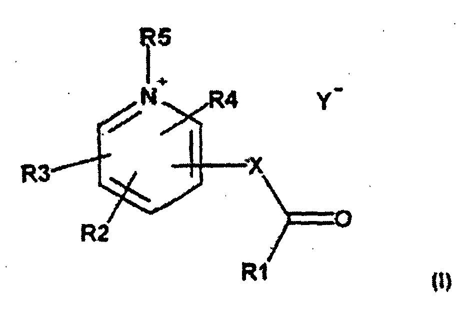 dans laquelle
R¹ représente un atome d'hydrogène, un groupe alkyle en C₁-C₄, un groupe aryle ou un groupe hétéroaryle,
R², R³ et R⁴ représentent, chaque fois indépendamment l'un de l'autre, un atome d'hydrogène, un atome d'halogène, un groupe alkyle en C₁-C₄, un groupe alcoxy en C₁-C₄, un groupe hydroxyalcoxy en C₁-C₄, un groupe hydroxyle, un groupe nitro, un groupe aryle, un groupe trifluorométhyle, un groupe amino qui peut être substitué par des groupes alkyle en C₁-C₄, ou un groupe acyle en C₁-C₄, deux des radicaux pouvant également former ensemble un noyau benzénique condensé,
R⁵ représente un groupe alkyle en C₁-C₄, un groupe aryle, un groupe aralkyle ou un groupe hétéroaryle,
X représente une liaison directe ou un groupe vinylène ou phénylène éventuellement substitué, et
Y⁻ représente un halogénure, un benzènesulfonate, un p-toluènesulfonate, un méthane-sulfonate, un trifluorométhane-sulfonate, un perchlorate, un sulfate, un hydrogénosulfate, un tétrachlorozincate ou un N-oxyde du groupe hétérocyclique, et
(B) au moins un composé comprenant un groupe amino primaire ou secondaire ou un groupe hydroxyle, choisi parmi le groupe comprenant des amines primaires ou secondaires aliphatiques ou aromatiques, des composés hétérocycliques azotés, des acides α-à ω-aminés, des oligopeptides constitués par un nombre de 2 à 9 acides aminés, et des composés hydroxylés aromatiques, et/ou au moins un composé contenant un groupe CH actif, le produit réactionnel des composants A et B pouvant également être présent.

3. Agent selon la revendication 2, **caractérisé en ce que** le composant A est choisi parmi le groupe constitué par des benzènesulfonates, des p-toluènesulfonates, des méthanesulfonates, des trifluorométhanesulfonates, des perchlorates, des sulfates, des chlorures, des bromures, des iodures et/ou des tétrachlorozincates du 4-formyl-1-méthylpyridinium, du 3-formyl-1-méthylpyridinium, du 2-formyl-1-méthylpyridinium, du 4-formyl-1-éthylpyridinium, du 2-formyl-1-éthylpyridinium, du 4-formyl-1-benzylpyridinium, du 2-formyl-1-benzylpyridinium, du 4-formyl-1,2-diméthylpyridinium, du 4-formyl-1,3-diméthylpyridinium, du 4-formyl-1-méthylquinolinium, du 2-formyl-1-méthylquinolinium, du 4-(2-formylvinyl)-1-méthyl-quinolinium, du 4-acétyl-1-méthyl pyridinium, du 2-acétyl-1-méthyl pyridinium, du 4-acétyl-1-quinolinium, du 4-acétyl-1-méthyl-quinolinium et du 4-(2-formylvinyl)-1-méthylpyridinium, du 2,6-dichloro-4-formyl-1-méthylpyridinium, du 2,6-diphényl-4-formyl-1-méthylpyridinium, du 4-benzoyl-1-méthylpyridinium, du 4-propionyl-1-méthylpyridinium, du 2-oximométhyl-1-méthylpyridinium, du N-oxyde du 4-pyridinecarboxaldéhyde et du N-méthylpyridoxal.

4. Agent selon la revendication 2 ou 3, **caractérisé en ce que** les composés du composant B sont choisis parmi le groupe constitué par des amines primaires ou secondaires du groupe constitué par la N-(2-hydroxyéthyl)-N-éthyldiamine, la N-(2-méthoxyéthyl)-diamine, la 2,3-, la 2,4-, la 2,5-dichloro-p-phénylènediamine, la 2-chloro-p-phénylènediamine, la N,N-bis-(2-hydroxyéthyl)-p-phénylènediamine, le dibromhydrate de la 2,5-dihydroxy-4-morpholinoaniline, le 2-, le 3-, le 4-aminophénol, la o-, la p-phénylènediamine, le 2,4-diaminophénoxyéthanol, le 2-(2,5-diaminophényl)-éthanol, le 2,5-diaminotoluène, le 2,5-diamino-phénol, le 2,5-diaminophénéthol, la 4-méthylaminoaniline, la 3-amino-4-(2'-hydroxyéthyloxy)-aniline, la 3,4-méthylènediamino-aniline, la 3,4-méthylènedioxyaniline, le 3-amino-2,4-dichlorophénol, le 4-méthylaminophénol, le 3,4-méthylènedioxyphénol, le 3-méthyl-4-aminophénol, le 2-méthyl-5-(2-hydroxyéthylamino)-phénol, le 6-méthyl-3-amino-2-chlorophénol, le 6-méthyl-3-amino-2-chloro-phénol, le 5-(2-hydroxyéthylamino)-4-méthoxy-2-méthylphénol, le 4-amino-2-aminométhylphénol, le 4-amino-2-hydroxyméthylphénol, le 3,4-méthylènedioxyphénol, le 1,3-diamino-2,4-diméthoxybenzène, l'acide 2-, 3-, 4-amino-benzoïque, -phénylacétique, l'acide 2,3-, 2,4-, 2,5-, 3,4-, 3,5-diamino-benzoïque, l'acide 4-, 5-amino-salicylique, l'acide 3-amino 4-hydroxybenzoïque, l'acide 4-amino-3-hydroxybenzoïque, l'acide 2-, 3-, 4-aminobenzènesulfonique, l'acide 3-amino-4-hydroxybenzène-sulfonique, l'acide 4-amino-3-hydroxy-naphtalènesulfonique, l'acide 6-amino-7-hydroxynaphtalène-2-sulfonique, l'acide 7-amino-4-hydroxynaphtalène-2-sulfonique, l'acide 4-amino-5-hydroxy-naphtalène-2,7-disulfonique, l'acide 3-amino-2-naphtoïque, l'acide 3-aminophtalique, l'acide 5-aminoisophtalique, le 1,3,5-, le 1,2,4-triaminobenzène, le tétrachlorhydrate du 1,2,4,5-tétraaminobenzène, le trichlorhydrate du 2,4,5-triaminophénol, le pentachlorhydrate de pentaaminobenzène, l'hexachlorhydrate d'hexaaminobenzène, le trichlorhydrate du 2,4,6-triaminorésorcinol, le sulfate du 4,5-diaminopyrocatéchol, le dichlorhydrate du 4,6-diaminopyrogallol, le sulfate du 3,5-diamino-4-hydroxypyrocatéchol, des anilines, respectivement des phénols aromatiques comprenant un radical aromatique supplémentaire, tels que le dichlorhydrate du 4,4'-diaminostilbène, l'acide 4,4'-diaminostilbène-2,2'-disulfonique, sel de sodium, le 4,4'-diaminodiphénylméthane, le 4,4'-diaminodiphényl-sulfure, le 4,4'-diaminodiphénylsulfoxyde, la 4,4'-diaminodiphényl-amine, l'acide 4,4'-diaminodiphénylamine-2-sulfonique, la 4,4'-diaminobenzophénone, l'éther 4,4'-diaminodiphénylique, le tétrachlorhydrate du 3,3',4,4'-tétraaminodiphényle, la 3,3',4,4'-tétraaminobenzophénone, le tétrachlorhydrate du 1,3-bis-(2,4-diaminophénoxy)-propane, le tétrachlorhydrate du 1,8-bis-(2,5-diaminophénoxy)-3,6-dioxaoctane, le 1,3-bis-(4-aminophénylamino)-propane, le 1,3-bis-(4-aminophénylamino)-2-propanol, le 1,3-bis-[N-(4-aminophényl)-2-hydroxyéthylamino]-2-propanol, le trichlorhydrate de la N,N-bis-[2-(4-aminophénoxy)-éthyl]-méthylamine, des composés hétérocycliques azotés choisis parmi le groupe constitué par la 2-, la 3-, la 4-aminopyridine, la 2-amino-3-hydroxypyridine, la 2,6-diamino-pyridine, la 2,5-diamino-pyridine, la 2,3-diamino-pyridine, la 2-diméthylamino-5-aminopyridine, la 3-amino-2-méthylamino-6-méthoxypyridine, la 2,3-diamino-6-méthoxypyridine, la 3,5-diamino-2,6-diméthoxypyridine, la 2,4,5-triaminopyridine, la 2,6-dihydroxy-3,4-diméthylpyridine, la 4,5,6-triaminopyrimidine, la 2-hydroxy-4,5,6-triaminopyrimidine, la 4-hydroxy-2,5,6-triaminopyrimidine, la 2,4,5,6-tétraaminopyrimidine, la 2-méthylamino-4,5,6-triamino-pyrimidine, la 2,4-diaminopyrimidine, la 4,5-diaminopyrimidine, la 2-amino-4-méthoxy-6-méthyl-pyrimidine, le 2,3,4-triméthylpyrrole, le 2,4-diméthyl-3-éthyl-pyrrole, le 3,5-diaminopyrazole, le 3,5-diamino-1,2,4-triazole, le 3-aminopyrazole, le 3-amino-5-hydroxypyrazole, la 2-, la 3-, la 8-amino-quinoléine, la 4-aminoquinaldine, l'acide 2-amino-nicotinique, l'acide 6-amino-nicotinique, la 5-aminoisoquinoléine, le 5-aminoindazole, le 6-aminoindazole, le 5-benzimidazole, le 7-amino-benzimidazole, le 7-amino-benzothiazole, la 2,5-dihydroxy-4-morpholinoaniline, ainsi que des dérivés d'indole et d'indoline, tels que le 4-, le 5-, le 6-, le 7-amino-indole, le 4-, le 5-, le 6-, le 7-hydroxyindole, le 5,6-dihydroxyindole, la 5,6-dihydroxyindoline et la 4-hydroxyindoline, et par, respectivement, les sels physiologiquement compatibles de ces composés, formés avec de préférence des acides inorganiques,
des composés hydroxylés aromatiques choisis parmi le groupe constitué par le 2-, le 4-, le 5-méthyl-résorcinol, le 2,5-diméthyl-résorcinol, le résorcinol, le 3-méthoxyphénol, le pyrocatéchol, l'hydroquinone, le pyrogallol, la phloroglucine, l'hydroxy-hydroquinone, le 2-, le 3-, le 4-méthoxyphénol, le 3-diméthylamino-phénol, le 2-(2-hydroxyéthyl)-phénol, le 3,4-méthylènedioxyphénol, l'acide 2,4-, 3,4-dihydroxy-benzoïque, - phénylacétique, l'acide gallique, l'acide 2,4,6-trihydroxybenzoïque, l'acétophénone, le 2-, le 4-chloro-résorcinol, le 1-naphtol, le 1,5-, le 2,3-, le 2,7-dihydroxy-naphtalène, l'acide 6-diméthylamino-4-hydroxy-2-naphtalène-sulfonique, l'acide 3,6-dihydroxy-2,7-naphtalènesulfonique, et des composés contenant un groupe CH actif choisis parmi le groupe constitué par l'iodure du 1,2,3,3-tétraméthyl-3H-indolium, le p-toluènesulfonate du 1,2,3,3-tétraméthyl-3-indolium, le méthane-sulfonate du 1,2,3,3-tétraméthyl-3-indolium, l'iodure du 2,3-diméthyl-benzothiazolium, le p-toluènesulfonate du 2,3-diméthyl-benzothiazolium, la rhodanine, l'acide rhodanine-3-acétique, l'iodure du 1-éthyl(méthyl)-2-quinaldinium, l'acide barbiturique, l'acide thio-barbiturique, l'acide 1,3-diméthyl(éthyl)thiobarbiturique, l'oxindole, la coumaranone et la 1-méthyl-3-phényl-2-pyrazolinone.

5. Agent selon l'une quelconque des revendications 2 à 4, **caractérisé en ce que** les composés des composants A et B sont contenus en une quantité de respectivement 0,03 à 65, en particulier de 1 à 40 millimoles, chaque fois rapportée à 100 g du colorant total.

6. Agent selon l'une quelconque des revendications 2 à 5, **caractérisé en ce qu'**on met en oeuvre des agents d'oxydation en une quantité de 0,01 à 6 % en poids, rapportés à la solution d'utilisation.

7. Agent selon l'une quelconque des revendications 2 à 6, **caractérisé en ce qu'**on met en oeuvre du peroxyde d'hydrogène à titre d'agent d'oxydation.

8. Agent selon l'une quelconque des revendications 2 à 7, **caractérisé en ce qu'**on met en oeuvre des agents tensioactifs anioniques, zwitterioniques et/ou non ioniques.

9. Procédé pour la coloration de fibres kératiniques, dans lequel on applique sur les fibres kératiniques un colorant contenant
(A) un ou plusieurs aldéhydes et une ou plusieurs cétones à radicaux onium répondant à la formule I ci-après ou leurs dérivés 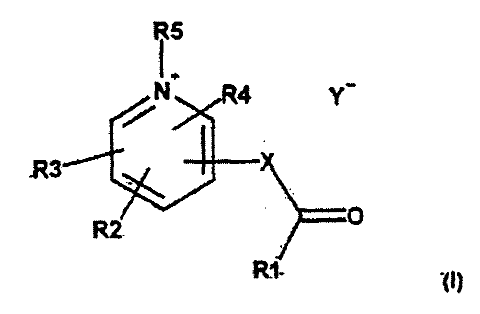 dans laquelle
R¹ représente un atome d'hydrogène, un groupe alkyle en C₁-C₄, un groupe aryle ou un groupe hétéroaryle,
R², R³ et R⁴ représentent, chaque fois indépendamment l'un de l'autre, un atome d'hydrogène, un atome d'halogène, un groupe alkyle en C₁-C₄, un groupe alcoxy en C₁-C₄, un groupe hydroxyalcoxy en C₁-C₄, un groupe hydroxyle, un groupe nitro, un groupe aryle, un groupe trifluorométhyle, un groupe amino qui peut être substitué par des groupes alkyle en C₁-C₄, ou un groupe acyle en C₁-C₄, deux des radicaux pouvant également former ensemble un noyau benzénique condensé,
R⁵ représente un groupe alkyle en C₁-C₄, un groupe aryle, un groupe aralkyle ou un groupe hétéroaryle,
X représente une liaison directe ou un groupe vinylène ou phénylène éventuellement substitué, et
Y⁻ représente un halogénure, un benzènesulfonate, un p-toluènesulfonate, un méthanesulfonate, un trifluorométhane-sulfonate, un perchlorate, un sulfate, un hydrogénosulfate, un tétrachlorozincate ou un N-oxyde du groupe hétérocyclique, et
(B) au moins un composé comprenant un groupe amino primaire ou secondaire ou un groupe hydroxyle, choisi parmi le groupe comprenant des amines primaires ou secondaires aliphatiques ou aromatiques, des composés hétérocycliques azotés, des acides aminés, des oligopeptides constitués par un nombre de 2 à 9 acides aminés, et des composés hydroxylés aromatiques, et/ou au moins un composé contenant un groupe CH actif,
le produit réactionnel des composants A et B pouvant également être présent,
ainsi que des constituants cosmétiques habituels, on laisse agir ledit colorant sur les fibres pendant un certain temps, habituellement pendant environ 30 minutes, avant de rincer une nouvelle fois et de procéder à un lavage en profondeur avec un shampooing.
